# EUROPEAN PATENT APPLICATION

(11) **EP 4 353 334 A1**
(43) Date of publication of application: **17.04.2024**
(21) Application number: 22807212.0
(22) Date of filing: 24.03.2022
(51) Int. Cl.: A63B 22/06, A61B 5/1455, A61B 5/22

(54) **OPTIMAL EXERCISE INTENSITY ESTIMATION METHOD, TRAINING METHOD, EXERCISE INSTRUCTION DEVICE, AND OPTIMAL EXERCISE INTENSITY ESTIMATION SYSTEM**

(30) Priority: 14.05.2021 JP 2021082111
(71) Applicant: Fancl Corporation, Kanagawa 231-8528 (JP)
(72) Inventor: ABE, Masatsugu, Yokohama-shi, Kanagawa 244-0806 (JP); UETO, Yuri, Yokohama-shi, Kanagawa 244-0806 (JP)
(74) Representative: Grünecker Patent- und Rechtsanwälte PartG mbB
(86) International application number: PCT/JP2022/013952
(87) International publication number: WO 2022/239507

(57) **Abstract**

An object is to provide a method, and a system, for estimating optimal exercise intensity, a training method that involves exercising at the optimal exercise intensity estimated by this estimation method, as well as an exercise instruction device, and an exercise instruction system, capable of indicating a workload for attaining an individual's optimal exercise intensity. As a solution, a method for estimating optimal exercise intensity is provided, which comprises: a step to give a ramped load to a subject so as to obtain a value of blood oxygen concentration (SpO₂) at each [predetermined] different workload, which is measured over a range that overlaps at least a part of 96 to 100%; and a step to determine a starting point of decline at which the measured value of blood oxygen concentration starts to show a declining trend as the workload increases; wherein the workload at the starting point of decline is estimated as an optimal exercise intensity for the subject.

## Description

### Technical Field

The present invention relates to an optimal exercise intensity estimation method, training method, exercise instruction device, and optimal exercise intensity estimation system.

### Background Art

A number of reports have shown that the greater a person's body strength demonstrated by muscle strength, cardiorespiratory capacity, etc., the better the person's health, higher his/her rate of survival, and lower his/her mortality will become (Non-patent Literatures 1, 2). Improving muscle strength, cardiorespiratory capacity, and other measures of body strength requires exercises of moderate or higher intensity; for example, one cannot improve his/her body strength by continuing exercises that lack intensity. While what exercises of moderate intensity are varies from one individual to another, it is known that the anaerobic threshold (AT) at which aerobic exercise changes to anaerobic exercise marks the onset of exercise of moderate intensity for all people.

The AT corresponds to the lactate threshold (LT) at which the lactate concentration in blood starts rising rapidly due to increased exercise intensity, or to the ventilation threshold (VT) at which the rate of increase in carbon dioxide in breath gas rises markedly due to increased exercise intensity. Traditionally, these thresholds are measured by two methods that are known as golden standard methods, including a testing method employing a breath gas analysis device and a method using blood lactate concentration. These methods require the subject to exercise at gradually increasing intensities until his/her maximum exercise intensity is reached, i.e., until he/she cannot exercise any further, which puts significant physical and mental burdens on the subject. Furthermore, measuring blood lactate concentration requires a blood draw that can be painful, while measuring carbon dioxide concentration in breath gas requires the subject to exercise while breathing through a mouthpiece connected to a breath gas measuring device-an expensive, specialty measuring device that needs monitoring by an expert (Patent Literature 1). Because of the needs for a specialty device and blood draw, AT measurement by golden standard methods can only be performed at hospitals, universities with sports education programs, or research institutes. To give the AT a broader role in the promotion of health, many researchers are studying alternative methods that offer accuracies comparable to the breath gas analysis method and blood lactate method; however, none of them are yet used in the fields of medicine/rehabilitation, sports, or promotion of health. Another method being studied involves obtaining the LT by measuring sweat lactate concentration; however, this method is not suitable for delivering accurate measurements because the lactate concentration in one's sweat is significantly influenced by the person's physical condition and diet.

### Background Art Literature

### Patent Literature

Patent Literature 1: Japanese Patent Laid-open No. 2018-134294

### Non-patent Literature

Non-patent Literature 1: Blair SN et al., Physical Fitness and All-cause Mortality. A Prospective Study of Healthy Men and Women. JAMA. 1989; 262 (17):2395-401.
Non-patent Literature 2: Jonathan Myers, Manish Prakash, Victor Froelicher, et al., Exercise Capacity and Mortality among Men Referred for Exercise Testing. Engl J Med 2002; 346:793-801.

### Summary of the Invention

### Problems to Be Solved by the Invention

An object of the present invention is to provide a method, and a system, for estimating an individual's optimal exercise intensity without requiring the person to exercise until his/her maximum exercise intensity is reached, i.e., until he/she cannot exercise any further, a training method that involves exercising at the optimal exercise intensity estimated by the estimation method, as well as an exercise instruction device, and an exercise instruction system, capable of indicating the workload for attaining the individual's optimal exercise intensity.

### Means for Solving the Problems

The means for achieving the object of the present invention are as follows.
1. A method for estimating optimal exercise intensity characterized in that it comprises:
   a step to give a ramped load to a subject so as to obtain a value of blood oxygen concentration (SpO₂) at each different workload, which is measured over a range that overlaps at least a part of 96 to 100%; and
   a step to determine a starting point of decline at which the measured value of blood oxygen concentration starts to show a declining trend as the workload increases;
   wherein the workload at the starting point of decline is estimated as an optimal exercise intensity for the subject.
2. The method for estimating optimal exercise intensity according to 1, characterized in that it is a method that measures a pulse rate simultaneously with the SpO₂ and determines the starting point of decline based on change in SpO₂ over time;
   wherein, once the pulse rate first exceeds a target pulse rate, the value of SpO₂ at the time the pulse rate first exceeded the target pulse rate is used as a reference value, and the measurement point of SpO₂ immediately before a range where the measured value of SpO₂ indicated a value lower than the reference value consecutively for 5 seconds or longer is determined as the starting point of decline.
3. The method for estimating optimal exercise intensity according to 1, characterized in that it is a method that measures the pulse rate simultaneously with the SpO₂ and determines the starting point of decline based on change in SpO₂ over time;
   wherein, once the pulse rate first exceeds the target pulse rate, the value of SpO₂ at the time the pulse rate first exceeded the target pulse rate is used as a reference value, and a point of intersection between a straight line connecting the measurement point of the highest value and measurement point of the lowest value, of SpO₂, in a range where the measured value of SpO₂ indicated a value lower than the reference value consecutively for 5 seconds or longer, and an approximate straight line before the range, is determined as the starting point of decline.
4. A method for estimating optimal exercise intensity characterized in that it comprises:
   a step to give a ramped load to a subject so as to obtain a value of blood oxygen concentration (SpO₂) at each different workload, which is measured over a range that overlaps at least a part of 96 to 100%, and measure the pulse rate simultaneously with the SpO₂; and
   a step to determine an inflection point at which the behavior of SpO₂/pulse rate changes as the workload increases;
   wherein the workload at the inflection point is estimated as an optimal exercise intensity for the subject.
5. A training method characterized in that exercise is performed at the optimal exercise intensity estimated by the estimation method according to any one of 1 to 4.
6. An exercise instruction device characterized in that it comprises:
   a storage means for storing biological information values at the optimal exercise intensity estimated by the estimation method according to any one of 1 to 4;
   a measurement means capable of measuring the biological information values;
   a computation means for calculating a workload by comparing the biological information values measured by the measurement means against the biological information values at the optimal exercise intensity; and
   an instruction means for indicating the workload calculated by the computation means.
7. The exercise instruction device according to 6, characterized in that:
   the measurement means is capable of measuring the blood oxygen concentration (SpO₂);
   the instruction means is capable of indicating the workload which is a workload used as a ramped load, based on information relating to the biological information values from the measurement means; and
   the computation means is capable of calculating a starting point of decline at which the measured value of blood oxygen concentration starts to show a declining trend as the workload increases.
8. The exercise instruction device according to 6, characterized in that:
   the measurement means is capable of measuring the blood oxygen concentration (SpO₂) and pulse rate;
   the instruction means is capable of indicating the workload which is a workload used as a ramped load, based on information relating to the biological information values from the measurement means; and
   the computation means is capable of calculating an inflection point at which the behavior of SpO₂/pulse rate changes as the workload increases.
9. The exercise instruction device according to any one of 6 to 8, characterized in that it is a wearable terminal.
10. A system for estimating optimal exercise intensity, characterized in that it comprises:
   a measurement part that measures the blood oxygen concentration (SpO₂) over a range that overlaps at least a part of 96 to 100%;
   an instruction part that indicates a workload that is used as a ramped load; and
   a computation part that calculates a starting point of decline at which the measured value of blood oxygen concentration starts to show a declining trend as the workload increases;
   wherein the workload at the starting point of decline is estimated as an optimal exercise intensity.
11. A system for estimating optimal exercise intensity characterized in that it comprises:
   a measurement part that measures the blood oxygen concentration (SpO₂) over a range that overlaps at least a part of 96 to 100%, while also simultaneously measures the pulse rate;
   an instruction part that indicates a workload that is used as a ramped load; and
   a computation part that calculates an inflection point at which the behavior of SpO₂/pulse rate changes as the workload increases;
   wherein the workload at the inflection point is estimated as an optimal exercise intensity.

### Effects of the Invention

According to the present invention, a measurement method that is simple but accurate and also offers excellent economy can be utilized to derive an individual's optimal exercise intensity, thereby contributing to promoting the person's health and extending his/her healthy lifespan. It is very important to derive exercise intensities for use in rehabilitation and everyday exercises for patients recuperating after surgery, as well as for seniors. Deriving optimal exercise intensities using conventional methods requires a blood draw as well as exercising until one cannot exercise any further, which presents significant physical and mental burdens. The present invention is a remarkable breakthrough not only for healthy individuals but also for patients recuperating after surgery, as well as for seniors, in that one's optimal exercise intensity can be obtained accurately without requiring such blood draw or exercising until one cannot exercise any further.

### Brief Description of the Drawings

[FIG. 1] A diagram showing how the SpO₂ and pulse rate of subject A changes over time during exercise under a ramped load.
[FIG. 2] A scatter plot of the SpO₂ and pulse rate of subject A during exercise under a ramped load.
[FIG. 3] A scatter plot of the SpO₂ and SpO₂/pulse rate of subject A during exercise under a ramped load.

### Mode for Carrying Out the Invention

### • Estimation Method and Estimation System

The first method for estimating optimal exercise intensity proposed by the present invention is characterized in that it comprises:
a step to give a ramped load to a subject so as to obtain a value of blood oxygen concentration (SpO₂) at each different workload, which is measured over a range that overlaps at least a part of 96 to 100%; and
a step to determine a starting point of decline at which the measured value of blood oxygen concentration starts to show a declining trend as the workload increases;
wherein the workload at the starting point of decline is estimated as an optimal exercise intensity for the subject.

The second method for estimating optimal exercise intensity proposed by the present invention is characterized in that it comprises:
a step to give a ramped load to a subject so as to obtain a value of blood oxygen concentration (SpO₂) at each different workload, which is measured over a range that overlaps at least a part of 96 to 100%, and measure the pulse rate simultaneously with the SpO₂; and
a step to determine an inflection point at which the behavior of SpO₂/pulse rate changes as the workload increases;
wherein the workload at the inflection point is estimated as an optimal exercise intensity for the subject.

It should be noted that, in the Specification, "A to B (A and B are numbers)" indicates a range of numerical values including the values of A and B, or specifically A or greater but no greater than B.

The blood oxygen concentration (SpO₂) is a ratio of binding of the red blood cell hemoglobin and oxygen in arterial blood. SpO₂ can be measured simply by wearing a measuring device (pulse oximeter) at the fingertip, over the wrist, etc. The method for estimating optimal exercise intensity proposed by the present invention is noninvasive and therefore puts minimal burden on the subject.

The first and second estimation methods have a step to give a ramped load to a subject so as to obtain a value of blood oxygen concentration (SpO₂) at each different workload, which is measured over a range that overlaps at least a part of 96 to 100%.

The exercise method to give a ramped load is not specifically limited, and a treadmill, bicycle ergometer, stepper, etc., may be adopted.

The measured value of SpO₂ should fall within a range that overlaps at least a part of 96 to 100%, such as 95 to 100%, 97 to 100%, 98 to 100%, 99 to 100%, 97 to 99%, etc. Decreasing the lower limit of the SpO₂ measuring range allows for more accurate estimation of optimal exercise intensity but increases exercise burden during measurement. For this reason, preferably a lower limit is set for each subject according to his/her sex, age, exercise habits or lack thereof, etc., and measurement is stopped once the measured value of SpO₂ drops below the set lower limit. In particular, lower blood oxygen concentrations (SpO₂) indicate greater burden on the subject, so this lower limit is preferably 95% or higher, or more preferably 96% or higher.

The SpO₂, etc., can be measured continuously, but since measurement is performed during exercise, the measuring devices may shift and prevent accurate measured values from being obtained. For this reason, preferably measured values obtained from intermittent measurements taken every 0.1 to 5 seconds or so are consolidated into average values over 1 to 30 seconds or so, and used as such. Also, since the workload gradually increases under a ramped load, the biological information values that are measured normally change only in one direction-for example, the SpO₂ changes only in the direction of decreasing, while the pulse rate changes only in the direction of increasing-and this allows for employment of, for example, processing that does not use values indicating change in the opposite-to-normal direction, or processing that does not use a value at the measurement point with a rate of deviation of, for example, 10% or greater from the average of values taken at around 2 to 5 points before it.

The second estimation method measures the pulse rate simultaneously with the SpO₂. It should be noted that the pulse rate can also be measured simultaneously with the SpO₂ under the first estimation method. Furthermore, the first and second estimation methods allow for measurement of one type, or two or more types, of biological information value(s) including the blood pressure, lactate concentration (in blood, in sweat), carbon dioxide concentration in breath gas, and so on. Of these, the pulse rate is preferred for simplicity of measurement.

Under the first and second estimation methods, preferably measurement is performed within a range of blood oxygen concentrations of 96% or higher and a range of pulse rates of 160 beats/less, so that burden on the subject can be reduced.

The first estimation method has a step to determine a starting point of decline at which the measured value of SpO₂ starts to show a declining trend as the workload increases.

The anaerobic threshold (AT) is a point at which aerobic exercise switches to anaerobic exercise, and at the AT oxygen concentration in the body starts to drop due to a lack of oxygen supply needed to continue exercising/producing energy. Accordingly, the starting point of decline at which the measured value of SpO₂ starts to show a declining trend approximates the AT.

Additionally, the workload at this starting point of decline approximates the workload at the AT, which allows the workload at the starting point of decline to be estimated as an optimal exercise intensity for the subject.

The second estimation method has a step to determine an inflection point at which the behavior of SpO₂/pulse rate changes as the workload increases.

As the workload increases, SpO₂ drops and pulse rate increases and therefore the SpO₂/pulse rate shows a dropping trend. That this SpO₂/pulse rate has an inflection point beyond which its slope increases, and that this inflection point approximates the anaerobic threshold (AT), are new insights gained by the inventors of the present invention.

The first estimation method may be implemented by, for example, a first system for estimating exercise intensity that comprises:
a measurement part that measures the blood oxygen concentration (SpO₂) over a range that overlaps at least a part of 96 to 100%;
an instruction part that indicates a workload that is used as a ramped load; and
a computation part that calculates a starting point of decline at which the measured value of blood oxygen concentration starts to show a declining trend as the workload increases;
wherein the workload at the starting point of decline is estimated as an optimal exercise intensity.

The second estimation method may be implemented by, for example, a second system for estimating optimal exercise intensity that comprises:
a measurement part that measures blood oxygen concentration (SpO₂) over a range that overlaps at least a part of 96 to 100%, while also simultaneously measures the pulse rate;
an instruction part that indicates a workload that is used as a ramped load; and
a computation part that calculates an inflection point at which the behavior of SpO₂/pulse rate changes as the workload increases;
wherein the workload at the inflection point is estimated as an optimal exercise intensity.

The first and second estimation systems may also have, besides the above, a storage part that stores the measured values, a communication part that exchanges data externally, and a display part that displays the contents of instructions, for example. Also, the storage part and computation part may be at least partially comprised of a cloud-based system whose processing takes place on an external server with which the estimation system communicates through the communication part. Furthermore, the first and second estimation systems may be a smartphone, smart watch, smart glasses, earpiece, or other wearable device that is made capable of implementing the aforementioned means by installing therein an application having a function to estimate optimal exercise intensity. The first and second estimation systems may be constituted by connecting a pulse oximeter or other measuring device having the instruction part, and a treadmill, bicycle ergometer, or other training machine found in gyms, etc., using a cable or wirelessly, so that while the measurement part (measuring device) measures the user's blood oxygen concentration, the instruction part indicates a speed, incline, resistance, etc., for the exercise machine, such as treadmill or bicycle ergometer, and instructs the user to adjust a workload to be a ramped load.

The first and second estimation systems obtain the blood oxygen concentration (SpO₂) over a range that overlaps at least a part of 96 to 100%, and the lower limit of the range is preferably 95% or higher, or more preferably 96% or higher. Since the first and second estimation systems measure the SpO₂ over a range that overlaps at least a part of 96 to 100%, where a preferred lower limit is 95% or higher, burden on the subject during measurement can be reduced. Also, the first and second estimation systems may be constituted by combining a pulse oximeter or other measuring device capable of measuring the blood oxygen concentration with a training machine capable of applying a ramped load, which means that these systems do not need to use any specialized measuring device under an expert's guidance and thus can be introduced to gyms, etc., for example.

Under the first estimation method and estimation system, the method for determining a starting point of decline from the measured value of SpO₂ is not specifically limited; however, methods 1-1 and 1-2 below can be mentioned, for example.

### • Method for determining starting point of decline 1-1 (also referred to as method 1-1)

A method that measures the pulse rate simultaneously with the SpO₂ and determines a starting point of decline based on change in SpO₂ over time, wherein the method is such that, once the pulse rate first exceeds the target pulse rate, the value of SpO₂ at the time the pulse rate first exceeded the target pulse rate is used as a reference value, and the measurement point of SpO₂ immediately before a range where the measured value of SpO₂ indicated a value lower than the reference value consecutively for 5 seconds or longer is determined as a starting point of decline.

### • Method for determining starting point of decline 1-2 (also referred to as method 1-2)

A method that measures the pulse rate simultaneously with the SpO₂ and determines a starting point of decline based on change in SpO₂ over time, wherein the method is such that, once the pulse rate first exceeds the target pulse rate, the value of SpO₂ at the time the pulse rate first exceeded the target pulse rate is used as a reference value, and the point of intersection between a straight line connecting the measurement point of the highest value and the measurement point of the lowest value, of SpO₂, within a range where the measured value of SpO₂ is lower than the reference value consecutively for 5 seconds or longer, and an approximate straight line determined before the aforesaid range, is set as a starting point of decline. If there are two or more measurement points giving the highest value or lowest value, one of such values obtained first chronologically is used as the measurement point of the highest value or lowest value.

Under the second estimation method and estimation system, the method for determining an inflection point of the calculated SpO₂/pulse rate is not specifically limited; however, method 2 below can be mentioned, for example.

### • Method for determining inflection point 2 (also referred to as method 2)

A method that measures the pulse rate simultaneously with the SpO₂ and, by using the pulse rate as an independent variable and the value obtained by dividing the SpO₂ by the pulse rate (SpO₂/pulse rate) as a dependent variable, and also based on a combination of regression line 1 covering the first measurement point to the nth (n≥2) measurement point and regression line 2 covering the (n+1)th measurement point to the Nth measurement point (N≥n+2), determines the point of intersection between regression lines 1 and 2 when the sum of the residual sums of squares of regression lines 1 and 2 becomes the smallest, as an inflection point.

It should be noted that residual mean squares may be used in place of residual sums of squares.

Under methods 1-1 and 1-2, the target pulse rate may be any value such as that used as a reference for aerobic exercise; for example, a value of target heart rate derived by the Karvonen formula {(220 - age - stationary heart rate) x (0.4 to 0.7) + stationary heart rate}, or a simplified version of it (220 - age) x 0.5 to 0.7, may be used, for example, or simply a value of around 120 to 130 beats per minute may be used.

Under methods 1-1 and 1-2, the number of seconds over which the measured value of SpO₂ indicates a value lower than the reference value (value of SpO₂ at the time the pulse rate first exceeded the target pulse rate) only needs to be 5 seconds or more. The longer the time in seconds, the lower the possibility becomes of determining a wrong starting point of decline based on measured values deviating from the trend; however, a longer measurement time increases physical burden on the subject. Accordingly, the lower limit of the time in seconds is preferably 8 seconds or more, or more preferably 10 seconds or more, while the upper limit of these seconds is preferably 60 seconds or less, or more preferably 50 seconds or less, or yet more preferably 40 seconds or less.

Also, preferably determination is made based on measured values from two or more consecutive multiple measurements to prevent misdetermination caused by a single measuring error. Specifically, when the measured values of SpO₂ are obtained as average values over 1 to 30 seconds or so, preferably the value obtained by multiplying the number of seconds over which to calculate a measured value (average value) of SpO₂ by (number of measurements - 1) is 5 seconds or longer, and, at the same time, two or more consecutive multiple measurement points deliver values lower than the reference value.

Under the first estimation method, no more exercise under ramped load is needed once this starting point of decline has been determined and the measurement can be terminated there, which means that there is no need to continue exercising until one cannot exercise any further, and consequently physical burden on the subject can be minimized.

Under method 2, where continuous exercising is required until an inflection point occurs, the measurement is continued preferably until 140 beats per minute is reached, or more preferably until 150 beats per minute is reached, or yet more preferably until 155 beats per minute is reached, or most preferably until 160 beats per minute is reached. However, this upper limit is preferably no more than 85%, or more preferably no more than 83%, or yet more preferably no more than 80%, of the expected maximum heart rate (220 - age) of the subject.

Under method 2, when regression line 1 derived from the measurement points from the first measurement point up to the mth, and regression line 2 derived from the measurement points from the (m+1)th measurement point up to the Mth measurement point, are used in combination to obtain the sum of the residual sums of squares of regression lines 1 and 2, if the sum becomes the smallest, the exercise is continued up to the (M+p)th measurement point; and when regression line 1, and regression line 2' derived from the measurement points from the (m+1)th measurement point up to the (M+p)th measurement point, are used in combination to obtain the sum of the residual sums of squares of them, and the sum becomes again the smallest, an inflection point may be determined there, and further exercise can be stopped. If the exercise is stopped prematurely as described above, M is preferably 5 or greater, while p is preferably 2 or greater. Additionally, the measurement time from the first to mth measurements, and that from the (m+1)th to Mth measurements, are each preferably 20 seconds or longer, or more preferably 30 seconds or longer, or yet more preferably 40 seconds or longer. Also, the measurement time from the Mth to (M+p)th measurements is preferably 10 seconds or longer, or more preferably 15 seconds or longer, or yet more preferably 20 seconds longer.

Under the second estimation method, exercise needs to be performed only within a prescribed range of pulse rates, and since there is no need to continue exercising until one cannot exercise any further, physical burden on the subject can be minimized.

### • Training Method

The training method proposed by the present invention is characterized in that exercise is performed at the optimal exercise intensity estimated by the aforementioned estimation method.

Using the estimation method proposed by the present invention, the workload at the starting point of decline or inflection point can be estimated as an optimal exercise intensity for the subject. Then, because this estimated optimal exercise intensity approximates the workload intensity at the AT, a training method that involves exercising at this optimal exercise intensity can efficiently improve one's body strength. On the other hand, one can expect hardly any improvement in his/her body strength by exercising continuously at exercise intensities lower than his/her optimal exercise intensity estimated by this estimation method, while exercising at exercise intensities higher than the optimal exercise intensity estimated by this estimation method may lead to injury or accumulation of fatigue.

### • Exercise Instruction Device

The exercise instruction device proposed by the present invention is characterized in that it comprises: a storage means for storing biological information values at the optimal exercise intensity estimated by the estimation method proposed by the present invention; a measurement means capable of measuring the biological information values; and an instruction means for indicating a workload by comparing the biological information values measured by the measurement means against the biological information values at the optimal exercise intensity.

The exercise instruction device proposed by the present invention may also have, besides the above, a storage means such as a memory, a communication means, a display means, a CPU or other computation means, a battery, and so on. Also, the storage means and computation means may be such that its processing takes place at least partially on an external server with which the exercise instruction device communicates through the communication parts.

The mode of the exercise instruction device proposed by the present invention is not specifically limited; for example, it may be built into a training machine or constitute an external terminal connected to a training machine, or it may be, for example, a smartphone, smart watch, smart glasses, earpiece, etc., that is made capable of implementing the aforementioned means by installing an application therein. Of these, preferably the exercise instruction device proposed by the present invention is a smart watch, smart glasses, or other wearable terminal.

The biological information values measured/stored by the exercise instruction device proposed by the present invention include the SpO₂, pulse rate, blood pressure, sweat lactate concentration, etc., of which one type or two or more types may be measured/stored. Of these, preferably the SpO₂ and pulse rate are measured and stored because of their ease of measurement.

The exercise instruction device proposed by the present invention can, by having the computation means compare biological information values at the estimated optimal exercise intensity stored by the storage means against biological information values measured by the measurement means, calculate the difference between current exercise intensity and optimal exercise intensity to calculate an optimal workload. Here, preferably the user is able to set a workload lower then, equal to, higher than, or the like, the optimal exercise intensity according to how he/she feels or his/her physical condition, etc., that day. Then, by exercising at the exercise intensity instructed by the exercise instruction device proposed by the present invention, the user can efficiently improve his/her body strength.

Furthermore, preferably the exercise instruction device proposed by the present invention is such that the measurement means can measure the SpO₂, the instruction means can indicate a workload that will be used as a ramped load, and the computation means can calculate a starting point of decline at which the measured value of blood oxygen concentration starts to show a declining trend as the workload increases.

Or, preferably the measurement means can measure the SpO₂ and pulse rate, the instruction means can indicate, based on information, a workload that will be used as a ramped load, and the computation means can calculate an inflection point at which the behavior of SpO₂/pulse rate changes as the workload increases.

Such exercise instruction device can estimate the user's optimal exercise intensity by virtue of its ability to indicate a workload that will be used as a ramped load and to calculate a starting point of decline at which the measured value of SpO₂ starts to show a declining trend or an inflection point at which the behavior of SpO₂/pulse rate changes. Therefore, assume that the body strength has been improved by exercising continuously for a while at the estimated optimal exercise intensity instructed by the exercise instruction device proposed by the present invention and thus the body strength can no longer be improved at this exercise intensity; even in this situation, by obtaining the latest optimal exercise intensity, exercise can be conducted at this exercise intensity representing a higher load.

### Examples

### (Examples)

### Workload application method 1

Equipment used: Ergometer
Load application method: Ramped load method
   Ergometer setting - Crank speed 120 bpm

The saddle is adjusted so that the subject's knee bends slightly when the pedal is at the lowest point.
Resting condition - Rest for 2 periods in a seated position (seated on the ergometer).
Warm-up condition - 5 minutes at 50 watts
Workload application condition - Ramped load increment 10 watts/min
Stopping condition - Load is lifted when one of the following conditions is satisfied:
   1) The subject can no longer continue exercising at 120 rpm due to fatigue in the lower limbs.
   2) The person in charge of testing determines that the test should be aborted.
Unit of load: watt

### Workload application method 2

Equipment used: Treadmill
Load application method: Ramped load method
   Resting condition - Rest for 2 periods in a seated position.
   Warm-up condition - 5 minutes at 5 km/h
   Workload application condition - Ramped load increment 1 km/h/min
   Stopping condition - Load is lifted when one of the following conditions is satisfied:
      1) The subject can no longer continue exercising due to fatigue in the lower limbs.
      2) The person in charge of testing determines that the test should be aborted.
   Unit of load: km/h

### (Measurement of SpO₂ and Pulse Rate)

The oxygen saturation and pulse rate were measured using a pulse oximeter (NELLCOR^{™} N-BSJ (Covidien Japan Inc.)).

The SpO₂ and pulse rate were measured at 4-second intervals and averaged every 20 seconds.

It was performed in a SpO₂ range of 96 to 100% and with the upper limit of pulse rate and target pulse rate set to 160 beats per minute and 120 beats per minute, respectively.

### (Breath Gas Parameters and Measurement of Heart Rate)

Measuring equipment: Breath gas analyzer AEROMONITOR AE-310SRC (Minato Medical Science Co., Ltd.), heart rate meter POLAR, heart rate sensor H10 N

Measuring method: Mixing chamber method

Measured items: Carbon dioxide production (VCO₂: ml/min), oxygen consumption (VO₂: ml/min), minute ventilation (VE), end-tidal partial pressure of oxygen tension (PETO₂), end-tidal partial pressure of carbon dioxide (PETCO₂), end-tidal oxygen concentration (ETO₂), end-tidal carbon dioxide concentration (ETCO₂), gas exchange rate (R=VCO₂/VO₂)*, ventilatory equivalent for oxygen (VE/VO2)*, ventilatory equivalent for carbon dioxide (VE/VCO₂)*, heart rate (HR: bpm) * Calculated value Data acquisition frequency: Once every 20 seconds

The VE/VO₂, and simultaneously SpO₂ and pulse rate, were measured on subjects A to C while a workload was given by workload application method 1 (ergometer), and on subjects D to F while a workload was given by workload application method 2 (treadmill). With every subject, measurement was performed until the person could no longer continue exercising, i.e., until the subject's maximum intensity was reached.

Table 1 shows, in the order of measurement, the results of SpO₂ and pulse rate taken at 15 measurement points that were arranged so that the target heart rates would appear at point no. 4. It should be noted that the measured values of subject B at point no. 10 were excluded because the pulse rate decreased from the value immediately before.

**[Table 1]**

| | A | | B | | C | | D | | E | | F | |
|---|---|---|---|---|---|---|---|---|---|---|---|---|
| No. | SpO₂ | Pulse rate | SpO₂ | Pulse rate | SpO₂ | Pulse rate | SpO₂ | Pulse rate | SpO₂ | Pulse rate | SpO₂ | Pulse rate |
| 1 | 99.0 | 118 | 100.0 | 119 | 97.0 | 117 | 100.0 | 111 | 100.0 | 114 | 98.4 | 110 |
| 2 | 98.2 | 117 | 100.0 | 118 | 97.0 | 117 | 100.0 | 113 | 100.0 | 114 | 98.4 | 112 |
| 3 | 98.8 | 120 | 100.0 | 118 | 97.0 | 119 | 100.0 | 114 | 100.0 | 116 | 98.8 | 118 |
| 4 | 99.0 | 121 | 99.6 | 124 | 97.4 | 121 | 100.0 | 121 | 100.0 | 121 | 98.8 | 122 |
| 5 | 99.0 | 122 | 100.0 | 124 | 97.0 | 124 | 100.0 | 129 | 100.0 | 125 | 99.0 | 126 |
| 6 | 99.0 | 126 | 99.8 | 127 | 97.0 | 127 | 100.0 | 146 | 100.0 | 127 | 99.0 | 133 |
| 7 | 99.0 | 127 | 99.6 | 129 | 97.4 | 128 | 99.8 | 150 | 99.6 | 133 | 99.2 | 138 |
| 8 | 99.0 | 127 | 99.6 | 132 | 97.2 | 131 | 99.6 | 152 | 100.0 | 141 | 99.0 | 139 |
| 9 | 98.6 | 130 | 99.6 | 132 | 96.6 | 131 | 97.8 | 149 | 100.0 | 140 | 99.0 | 143 |
| 10 | 98.0 | 133 | | | 96.8 | 133 | 98.0 | 153 | 99.4 | 147 | 98.6 | 145 |
| 11 | 98.4 | 135 | 99.4 | 135 | 96.0 | 135 | 98.4 | 154 | 99.6 | 150 | 97.8 | 146 |
| 12 | 98.4 | 136 | 99.0 | 138 | 97.0 | 139 | 99.0 | 158 | 99.2 | 148 | 98.0 | 151 |
| 13 | 98.0 | 139 | 99.0 | 139 | 96.6 | 141 | 99.0 | 152 | 99.6 | 157 | 99.0 | 159 |
| 14 | 98.0 | 142 | 99.2 | 141 | 96.2 | 140 | 98.0 | 159 | 100.0 | 160 | 99.0 | 159 |
| 15 | 98.0 | 145 | 98.4 | 149 | 96.6 | 145 | 97.4 | 155 | 100.0 | 162 | 99.0 | 164 |

### • Determination of AT by GS Method

By plotting the data taken every 20 seconds from the start of ramped load application until it was stopped, the point at which the VE/VCO₂ did not increase but the VE/VO₂ did, with respect to the exercise intensity, was determined as the AT.

### • Determination of Starting Point of Decline by Method 1-1

Under method 1-1, the value of SpO₂ when the pulse rate first exceeded the target pulse rate (120 beats per minute) was used as a reference value, and the measurement point of SpO₂ immediately before a range where the measured value of SpO₂ indicated a value lower than this reference value for 40 seconds (20 seconds x (3-1)) was determined as starting point of decline 1.

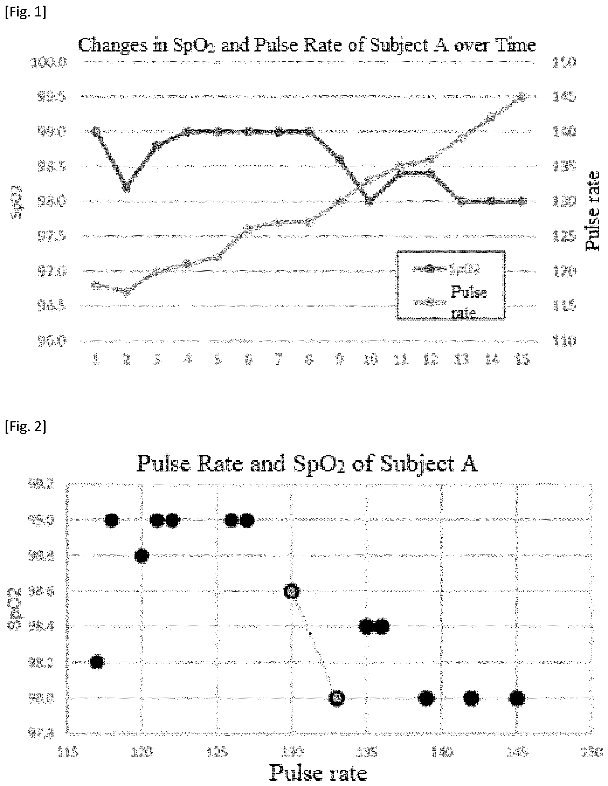

FIG. 1 shows how the SpO₂ and pulse rate of subject A changed over time. In the case of subject A, for example, whose reference value is 99 corresponding to the SpO₂ at point no. 5, the SpO₂ indicated a value lower than this reference value for 40 seconds from point nos. 9 to 11, and therefore the measurement point immediately before this, or point no. 8, was determined as a starting point of decline. Then, the workload at this starting point of decline (workload giving a heart rate of 127) was estimated as an optimal exercise intensity for subject A.

According to method 1-1, subject A needs to exercise only until point no. 11 to allow for estimation of optimal exercise intensity, upon which the measurement (exercise) can be terminated.

### • Determination of Starting Point of Decline by Method 1-2

Under method 1-2, the value of SpO₂ at the time the pulse rate first exceeded the target pulse rate (120 beats per minute) was used as a reference value, and the point of intersection between a straight line connecting the measurement point of the highest value and the measurement point of the lowest value, of SpO₂, within a range where the measured value of SpO₂ was lower than the above reference value for 40 seconds (20 seconds x (3-1)), and an approximate straight line determined before the aforesaid range (from 120 beats up to immediately before the range), was set as starting point of decline 2.

FIG. 2 shows a scatter plot of the SpO₂ and pulse rate of subject A. With subject A, after the pulse rate had first exceeded the target heart rate, the pulse rate increased as the workload increased. In the case of subject A, for example, whose reference value is 99 corresponding to the SpO₂ at point No. 5, the values of the SpO₂ from point Nos. 9 to 11 for 40 seconds were lower than this reference value, wherein the highest point was No. 9 and the lowest point was No. 10. The point of intersection (SpO₂, pulse rate = 99, 128) between a straight line connecting these two points, and an approximate straight line derived from point Nos. 4 to 8 (all of their SpO₂ being 99), i.e., after exceeding 120 beats immediately before this range, was set as a starting point of decline. Then, the workload at this starting point of decline (workload giving a heart rate of 128) was estimated as an optimal exercise intensity for subject A.

According to method 1-2, subject A needs to exercise only until point No. 11 to allow for estimation of optimal exercise intensity, upon which the measurement (exercise) can be stopped.

### • Determination of Inflection Point by Method 2

Under method 2, the pulse rate was measured simultaneously with the SpO₂, and by using the pulse rate as an independent variable and the value obtained by dividing the SpO₂ by the pulse rate as a dependent variable, regression line 1 derived from the first measurement point up to the Nth (N≥2) measurement point and regression line 2 derived from the N+1th measurement point up to the last measurement point are used in combination to determine the sum of the residual sums of squares of two regression lines 1 and 2, and if the sum became the smallest, the point of intersection between regression lines 1 and 2 was set as an inflection point.

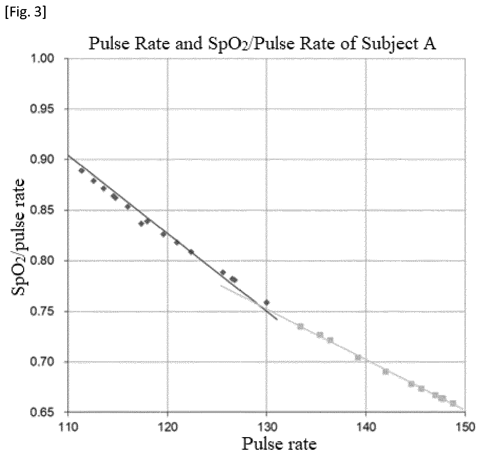

FIG. 3 shows a scatter plot of the pulse rate at and after 110 (including point Nos. 1 to 15), and SpO₂/pulse rate, of subject A. It should be noted that subject A could no longer continue exercising after 2 minutes of exercise upon point No. 15, at which time the subject's SpO₂ was 98.0 and pulse rate was 148. When these plotted points were divided into two and the sum of the residual sums of squares of the two respective regression lines was obtained, the sum of the residual sums of squares became the smallest when the points were divided into the measurement points up to No. 9 and the measurement points from No. 10 and thereafter, and therefore the point of intersection between these two regression lines (pulse rate, SpO₂/pulse rate = 127, 0.78) was set as an inflection point. Then, the workload at this inflection point (workload giving a heart rate of 127) was estimated as an optimal exercise intensity for subject A.

According to method 2, as it is applied to subject A, the sum of the residual sums of squares becomes the smallest even when the measurement points extended until 140 beats per minute was reached (point No. 13)wherein the measurement points were divided into the point up to No. 9 and point Nos. 10 to 13, and since this allows for estimation of an optimal exercise intensity equivalent to when exercise was continued until the subject could not exercise any further (2 more minutes past point No. 15), the measurement (exercise) can be stopped at point No. 13.

Table 2 shows the optimal exercise intensities estimated by the respective methods mentioned above, together with the optimal exercise intensities measured by the golden standard method (GS method) based on breath gas analysis.

**[Table 2]**

| Pulse Rates at Optimal Exercise Intensities | | | | | | |
|---|---|---|---|---|---|---|
| | A | B | C | D | E | F |
| GS method | 115 | 139 | 134 | 145 | 139 | 146 |
| Method 1-1 | 127 | 132 | 128 | 146 | 140 | 143 |
| Method 1-2 | 128 | 134 | 131 | 150 | 144 | 144 |
| Method 2 | 127 | 132 | 137 | 134 | 138 | 147 |

It was confirmed that optimal exercise intensities approximating the optimal exercise intensities measured by the conventional gold standard method (GS method) based on breath gas analysis can be estimated according to the estimation methods conforming to the present invention.

In the case of subject A, for example, an optimal exercise intensity can be estimated by exercising until measurement point no. 11 according to methods 1-1 and 1-2, upon which the measurement (exercise) can be terminated; according to method 2, on the other hand, an optimal exercise intensity can be estimated by exercising until measurement point no. 13. The estimation methods conforming to the present invention eliminate the need to exercise to the limit (until the subject cannot exercise any further), and since they do not require a blood draw, either, physical and mental burdens are minimized.

## Claims

1. A method for estimating optimal exercise intensity, **characterized by** comprising:
a step to give a ramped load to a subject so as to obtain a value of blood oxygen concentration (SpO₂) at each different workload, which is measured over a range that overlaps at least a part of 96 to 100%; and
a step to determine a starting point of decline at which the measured value of blood oxygen concentration starts to show a declining trend as the workload increases;
wherein the workload at the starting point of decline is estimated as an optimal exercise intensity for the subject.

2. The method for estimating optimal exercise intensity according to claim 1, **characterized by** being a method that measures a pulse rate simultaneously with the SpO₂ and determines the starting point of decline based on change in SpO₂ over time;
wherein, once the pulse rate first exceeds a target pulse rate, the value of SpO₂ at a time the pulse rate first exceeded the target pulse rate is used as a reference value, and a measurement point of SpO₂ immediately before a range where the measured value of SpO₂ indicated a value lower than the reference value consecutively for 5 seconds or longer is determined as the starting point of decline.

3. The method for estimating optimal exercise intensity according to claim 1, **characterized by** being a method that measures a pulse rate simultaneously with the SpO₂ and determines the starting point of decline based on change in SpO₂ over time;
wherein, once the pulse rate first exceeds a target pulse rate, the value of SpO₂ at a time the pulse rate first exceeded the target pulse rate is used as a reference value, and a point of intersection between a straight line connecting a measurement point of a highest value and a measurement point of a lowest value, of SpO₂, in a range where the measured value of SpO₂ indicated a value lower than the reference value consecutively for 5 seconds or longer, and an approximate straight line before the range, is determined as the starting point of decline.

4. A method for estimating optimal exercise intensity, **characterized by** comprising:
a step to give a ramped load to a subject so as to obtain a value of blood oxygen concentration (SpO₂) at each different workload, which is measured over a range that overlaps at least a part of 96 to 100%, and measure a pulse rate simultaneously with the SpO2; and
a step to determine an inflection point at which a behavior of SpO₂/pulse rate changes as the workload increases;
wherein the workload at the inflection point is estimated as an optimal exercise intensity for the subject.

5. A training method **characterized in that** exercise is performed at the optimal exercise intensity estimated by the estimation method according to any one of claims 1 to 4.

6. An exercise instruction device **characterized by** comprising:
a storage means for storing biological information values at the optimal exercise intensity estimated by the estimation method according to any one of claims 1 to 4;
a measurement means capable of measuring the biological information values;
a computation means for calculating a workload by comparing the biological information values measured by the measurement means against the biological information values at the optimal exercise intensity; and
an instruction means for indicating the workload calculated by the computation means.

7. The exercise instruction device according to claim 6, **characterized in that**:
the measurement means is capable of measuring a blood oxygen concentration (SpO₂);
the instruction means is capable of indicating the workload which is a workload used as a ramped load, based on information relating to the biological information values from the measurement means; and
the computation means is capable of calculating a starting point of decline at which the measured value of blood oxygen concentration starts to show a declining trend as the workload increases.

8. The exercise instruction device according to claim 6, **characterized in that**:
the measurement means is capable of measuring a blood oxygen concentration (SpO₂) and a pulse rate;
the instruction means is capable of indicating the workload which is a workload used as a ramped load, based on information relating to the biological information values from the measurement means; and
the computation means is capable of calculating an inflection point at which a behavior of SpO₂/pulse rate changes as the workload increases.

9. The exercise instruction device according to any one of claims 6 to 8, **characterized by** being a wearable terminal.

10. A system for estimating optimal exercise intensity, **characterized by** comprising:
a measurement part that measures a blood oxygen concentration (SpO₂) over a range that overlaps at least a part of 96 to 100%;
an instruction part that indicates a workload that is used as a ramped load; and
a computation part that calculates a starting point of decline at which the measured value of blood oxygen concentration starts to show a declining trend as the workload increases;
wherein the workload at the starting point of decline is estimated as an optimal exercise intensity.

11. A system for estimating optimal exercise intensity, **characterized by** comprising:
A measurement part that measures a blood oxygen concentration (SpO₂) over a range that overlaps at least a part of 96 to 100%, and also simultaneously measures a pulse rate;
an instruction part that indicates a workload that is used as a ramped load; and
a computation part that calculates an inflection point at which a behavior of SpO₂/pulse rate changes as the workload increases;
wherein the workload at the inflection point is estimated as an optimal exercise intensity.
